(19) Europäisches Patentamt
European Patent Office
Office européen des brevets

(11) **EP 2 422 848 A1**

(12) **EUROPEAN PATENT APPLICATION**

(43) Date of publication:
29.02.2012 Bulletin 2012/09

(51) Int Cl.:
*A61P 35/00* (2006.01)   *A61K 31/404* (2006.01)
*A61K 31/513* (2006.01)   *A61K 31/353* (2006.01)

(21) Application number: **10173253.5**

(22) Date of filing: **18.08.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(71) Applicant: **Grindeks, a joint stock company**
**1057 Riga (LV)**

(72) Inventors:
• **Kalvins, Ivars**
**Riga LV-1006 (LV)**
• **Stonans, Ilmars**
**Riga LV-1057 (LV)**
• **Sestakova, Irina**
**Riga LV-1006 (LV)**
• **Domracova, Ilona**
**Riga LV-1006 (LV)**
• **Jascenko, Elina**
**Riga LV-1006 (LV)**
• **Bridane, Veronika**
**Riga LV-1006 (LV)**
• **Kanepe, Iveta**
**Riga LV-1006 (LV)**

(54) **Composition of tegafur, Indole-3-carbinol and either catechin, kaempherol, myricetin or luteolin for potentiating antitumour effect and for treating tumours**

(57) Novel pharmaceutical compostion for treating cancer, preferably malignant cancer by way of enteral use have been described, which contain Indole-3-carbinol and one of natural flavonoid derivative, preferably Catechin, Kaempferol, Myricetin, Luteolin and a cancer chemotherapeutic agent TEGAFUR.

EP 2 422 848 A1

**Description**

Technical Field

The present invention relates to a novel composition of TEGAFUR and Indole-3-carbinol (13C) and natural flavonoid derivate, preferably Catechin, Kaempherol, Myricetin or Luteolin for potentiating anti-tumour effect and for treating tumours; in particular, relates to the unexpected synergism of a combination of an Indole-3-carbinol and natural flavonoid derivatives, such as Catechin, Kaempherol, Myricetin or Luteolin compounds and the anti-tumour agent TEGAFUR in the treatment of cancer; and to processes for preparing the composition and its use for chemotherapy of tumours, especially malignant tumours.

Background Art

More than 11 million people are diagnosed with cancer every year. It is estimated that there will be 16 million new cases every year by 2020. Cancer causes 7 million deaths every year - or 12.5% of deaths worldwide.
Therefore, a great number of investigations, developments and research works have been carried out to find anticancer agents, which can be used in chemotherapy of malignant tumours. There are known various anticancer agents and results of cancer treatment improve with every year, nevertheless the amount of effective dose of anticancer agent for treatment is still high.
One of such anticancer agents is TEGAFUR. TEGAFUR, i.e. 1-(2-tetrahydrofuryl)-5-fluorouracil, is disclosed in GB 1 168 391 (INST ORGANOCHESKOGO SINTEZA, published 22.10.1969) as a prodrug of 5-Fu, i.e. 5-fluorouracil, and proved active in the management of metastatic colorectal cancer. TEGAFUR is metabolized to 5-fluorouracil (5-Fu) in vivo, predominantly in the liver, and has been reported to be less toxic and to have a higher therapeutic index CAO than 5-fluorouracil prodrug. It plays a role in anabolic and catabolic pathway modulation in therapy of colorectal cancer (see "Cancer Research" 1995, vol.1, p. 839-845).
US 4 328 229 (TAIHO PHARMACEUTICAL, published 04.05.1982) discloses an anti-cancer composition containing 1-(2-tetrahydrofuryl)-5-fluorouracil (TEGAFUR) and uracil. The composition is used for delivery of 5-fluorouracil to a tumour which is sensitive to 5-fluorouracil in a warm-blooded animal. It is disclosed therein that the composition can be coadministered in a variety dosage forms including an oral dosage form.
Indole-3-Carbinol (13C) is a breakdown product of the glucosinolate glucobrassicin, also known as indole-3-glucosinolate. Glucosinolates are beta-thioglucoside N-hydroxysulfates, which are primarily found in cruciferous vegetables (cabbage, broccoli sprouts, Brussels sprouts. Indole-3-carbinol may have cancer chemopreventive activity, it may also have anticarcinogenic, antioxidant and anti-atherogenic acitivities, see http://www.pdrhealth.com/drug_info/nmdrugprofiles/nutsupdrugs/ind 031 5.shtml 11.07.2007
Because of its anticarcinogenic effects in experimental animals and humans, 13C has received special attention as a possible chemopreventive agent. 13C has also been found to inhibit the growth of various cancer cells and possibly to inhibit breast cancer invasion and migration (see Sarkar FH, Li Y. "Indole-3-carbinol and prostate cancer", published in "The Journal of Nutrition" 2004, vo1.134, No.12, p.3493S-3498S.
US 2007099844 discloses that the combination of indoleamine 2,3-dioxygenase (IDO) inhibitor with a chemotherapeutic agent act synergistically to suppress tumour growth. As one of IDO is Indole-3-carbinol (13C), and as a one of chemotherapeutic agent is 5-fluorouracil (5-Fu).
Myricetin is a naturally-occurring flavonoid found in many grapes, berries, fruits, vegetables, herbs, as well as other plants. Walnuts are a rich dietary source. Myricetin has antioxidant, antibacterial and antiviral activities and may have anti-inflammatory activity. In test tubes studies, Myricetin has shown anti-tumour activity.
Luteolin is a flavonoid and more specifically a flavone. It is thougjt to play an important role in the human body as an antioxidant, a free radical scavenger, and an immune system modulator. These characteristics of luteolin are also believed to play an important part in the prevention of cancer. Multiple research experiments describe luteloin as a biochemical agent that can dramatically reduce inflammation and the symptoms of septic shock.
Luteolin (3,4,5,7-tetrahydroxy flavone) an important member of the flavonoid family, is present in various fruits and vegetables and has contributed to the antioxidant activity of artichoke leaf extract on reactive oxygen species in human leucocytes.
Luteolin is also reported to have anti-inflammatory properties and mediates its action by inhibiting of nitric oxide production. Luteolin has anti-allergic properties [18], and a recent report establishes luteolin as a potent inhibitor of human mast cell activation through the inhibition of protein kinase C activation and Ca#+ in ux. Luteolin exerts growth inhibitory effects onNK}Ly ascites-tumour-cell cultures in .i.o [20]. The growth and metabolism of human leukaemic CEM-C1 and CEM-C7 cell lines are also inhibited by luteolin
Catechins are polyphenolic antioxidant plant metabolites, flavonoids called flavan-3-ols.
Catechins have potent antioxidant and cancer chemopreventive properties. These compounds are found in a variety of plants and are present in particularly high amounts in tea leaves, published in "Cancer research", 2001, No. 61, p.118-125.

High levels of monomeric (+)-catechin are found in the skin and seeds of fruits such as apples and grapes. Red wine and chocolate also are significant sources (+)-catechin. In keeping with the ability of phenolic antioxidants to induce the expression of phase II detoxifying enzymes in mammalian cells, (+)-catechins produces antimutagenic effects, published in "Cancer research" 2001, no.61, p.118-125.

Kaemferol is a flavone-ring-containing plant pigment found in many angiosperms (flowering plants). Kaempherol is a natural flavonoid which has been isolated from tea, broccoli, Delphinium, Witch-hazel, grapefruit and other plant sources. Kaempherol consumption in tea and broccoli has been associated with reduced risk of heart disease.

Disclosure of Invention

Technical problem

In malignant tumour treatment different kind of pharmaceutical compositions are used, including pharmaceutical compositions of TEGAFUR, bur just only partial remission of malignant tumour was obtained.

As well as composition which is disclosed in WO 2005105086 (TAIHO PHARMACEUTICAL CO LTD) 11.10.2005 said that it reducing the gastrointestinal toxicity of TEGAFUR and improving tolerability of 5-Fu, but still only partial remission of tumour is developed.

The further aim of present invention is to developed effective pharmaceutical composition that can be used in malignant tumour treatment for achieving increased remission of tumours.

Technical solution

The present invention is directed to a composition for treating a tumour, especially a malignant tumour in an individual who need such treatment, comprising the administration to said individual of a pharmacologically effective dose of derivatives of natural compounds and the cancer chemotherapeutic agent TEGAFUR.

While attempting to develop a pharmaceutical composition for malignant tumour treatment having an essentially lower toxicity, we unexpectedly found that doses adequate to the therapeutic ones used in the clinical use of pharmaceutical compositions containing TEGAFUR [1-(2-tetrahydrofuryl)-5-fluorouracil], a drug for treating a malignant tumour, the co-administration or combined use in pharmaceutical composition of a compound of the Indole-3-carbinol and one of the natural flavonoid derivative, preferably Catechin, Myricetin, Kaempherol, Luteolin, leads to advantageous effects such as:

■ The use of a composition of TEGAFUR and Indole-3-carbinol and one of the natural flavonoid derivates, preferably Catechin, Myricetin, Kaempherol, or Luteolin, that allows efficacy increase growth inhibition of S-180 ascites tumour;
■ Combination of Tegafur, Indole-3-carbinol and a natural flavonoid derivate showed remarkable synergism between the components in the treatment of tumour;

There is no particular restriction on the unit dosage from which can be adopted for the anti-tumour composition of the invention in the treatment of malignant tumours in mammals. The unit dosage form is selected according to the purpose of treatment. Examples thereof are oral dosage form such as tablets, coated tablets, pills, powders, granules, capsules, solutions, suspensions, emulsions, etc. and parenteral dosage forms such as suppositories, ointments, plasters, etc. These dosage dorms can be manufactured by convention pharmaceutical procedures known in this field. As the carrier for shaping into the form of tablets, there can be employed various excipients such as lactose, sucrose, sodium, glucose, urea, starch, calcium carbonate, kaolin, crystalline cellulose, silicic acid, etc.; binders such as simple syrup, glucose solution, starch solution, gelatine solution, carboxymethylcellulose, shellac, methylcellulose, potassium phosphate, polyvinylpyrrolidone, etc,; disintegrators such as dry starch, sodium alginate, agar powder, laminaran powder, sodium hydrogen carbonate, calcium carbonate, polyoxyethylene-sorbitan fatty acid esters, sodium lauryl sulphate, stearic acidmonoglyceride, starch, lactose, etc.; antidisintegrators such as sucrose, stearic acid, cacao butter, hydrogenated oil etc.; absorption promotors such as quaternary ammonium bases, sodium lauryl sulphate, etc.; humectants such as glycerol, starch, etc.; adsorbents such as starch, lactose, kaolin, bentonite, colloidal silicic acid, etc.; and lubricants such as purified talc, stearic acid salts. Boric acid powder, polyethylene glycol, etc. Where necessary, the tablets may be in the form of coated tablets such as sugar-coated tablets, gelat-coated tablets, enteric-coated tablets, film-coated tablets, or double or multi-layer tablets, etc.

The carrier for shaping into the form of pills includes, for example, various excipients such as glucose, lactose, starch, cacao butter, hardened vegetable oil, kaolin, talc, etc.; and disintegrators such as laminarian, agar, etc. The carrier for shaping into the form of suppositories includes, for example, polyethylene glycol, cacao butter, higher alcohols, esters of higher alcohols, gelatine, semi-synthetic glycerides, etc.

Capsules are manufactured by mixing Indole-3-carbinol and natural flavonoid derivative, preferably, Catechin, Myricetin, Kaempherol or Luteolin with TEGAFUR, with any of the carriers mentioned above and encapsulating the mixture in hard gelatine capsule, soft capsule or other capsules.

For manufacturing in the form of pastes, creams and gels, there is employed diluent such as, for example, white petrolatum, paraffin, glycerol, cellulose derivatives, polyethylene glycols, silicone, bentonite, etc.

Best Mode for Carrying Out the Invention

The present invention in the following will be described in more detail with reference to pharmacological tests and examples illustrating the preparation of the anti-tumour effect-potentiating compositions of the invention comprising Indole-3-carbinol and one of natural flavonoid derivative, preferably Catechin, Myricetin, Kaempherol or Luteolin and TEGAFUR.

PHARMACOLOGICAL TEST

Experimental example

Experiments in vivo were carried out on mice sarcoma S-180 ascites form.

The anti-tumour activity of the pharmaceutical composition of TEGAFUR and Indole-3-carbinol and one of natural flavonoid derivative's was determined by introducing them into ICR mice of initial weight 20-23 g during a fortnight. Throughout the experiment, the mice were kept under standard laboratory conditions at a temperature of $22\pm2$ °C, relative humidity 55 $\pm$15%, ventilation - 15-20 changes of air amount/hour and a 12 hours light/darkness cycle, and were fed with standard laboratory animal food.

Mice sarcoma S-180 transplanted intraperitoneally to ICR mice in amount 5 x $10^6$ cell/mouse.

Then, 24 hours later, orally administration of compounds was started once a day at the 1st, 3rd, 5th, 7th, 9th, 11th and 13th days.

The compound efficacy was expressed as the percentage of growth inhibition of S-180 ascites tumour, calculated using the equation:

Growth inhibition of S -180 ascites tumour(%) $= 100 - \left( \dfrac{T}{C} \times 100 \right)$

wherein C - mean weight (g) of the control group and T - mean weight (g) of the experimental group.

The results of Table 1 show the growth inhibition of S-180 ascites tumour at 13th day after the transplantation of tumour in mice.

Table 1

| Nr. | Drug | Dosage (mg/kg) | Growth inhibition of S-180 ascites tumour, % |
|---|---|---|---|
| 1 | TEGAFUR | 25 | 9 |
| 2 | Indole-3-carbinol | 25 | -20 |
| 3 | Catechin | 100 | 9 |
| 4 | TEGAFUR+Indole-3-carbinol+ Catechin | 25+25+100 | 33 |
| 5 | TEGAFUR | 25 | 9 |
| 6 | Indole-3-carbinol | 75 | -13 |
| 7 | Catechin | 300 | 6 |
| 8 | TEGAFUR+Indole-3-carbinol+ Catechin | 25+75+300 | 59 |
| 9 | TEGAFUR | 25 | 9 |
| 10 | Indole-3-carbinol | 200 | 8 |
| 11 | Kaempferol | 100 | -9 |
| 12 | TEGAFUR+Indole-3-carbinol+ Kaempferol | 25+200+100 | 54 |
| 13 | TEGAFUR | 25 | 9 |
| 14 | Indole-3-carbinol | 100 | 7 |
| 15 | Myricetin | 100 | -6 |

(continued)

| Nr. | Drug | Dosage (mg/kg) | Growth inhibition of S-180 ascites tumour, % |
|-----|------|----------------|---------------------------------------------|
| 16 | TEGAFUR+Indole-3-carbinol+ Myricetin | 25+100+100 | 44 |
| 17 | TEGAFUR | 25 | 9 |
| 18 | Indole-3-carbinol | 200 | 8 |
| 19 | Myricetin | 200 | 3 |
| 20 | TEGAFUR+Indole-3-carbinol+ Myricetin | 25+200+200 | 73 |
| 21 | TEGAFUR | 25 | 9 |
| 22 | Indole-3-carbinol | 25 | -20 |
| 23 | Luteolin | 6.25 | 7 |
| 24 | TEGAFUR+Indole-3-carbinol+ Luteolin | 25+25+6.25 | 53 |
| 25 | TEGAFUR | 25 | 9 |
| 26 | Indole-3-carbinol | 100 | 7 |
| 27 | Luteolin | 25 | 12 |
| 28 | TEGAFUR+Indole-3-carbinol+ Luteolin | 25+100+25 | 30 |
| 29 | TEGAFUR | 25 | 9 |
| 30 | Indole-3-carbinol | 200 | 8 |
| 31 | Luteolin | 50 | -39 |
| 32 | TEGAFUR+Indole-3-carbinol+ Luteolin | 25+200+50 | 58 |

As it is shown in Table 1 combinations of Tegafur, Indole-3-carbinol and one of natural flavonoid derivative showed remarkable synergism between the components and allows efficacy increase growth inhibition of S-180 ascites tumour.

Formulation Example 1: Granules

A possible pharmaceutical composition for oral use exemplifying but not exhausting the present invention is the following formulation of granules manufacture:

| | Example1 | Example2 | Example3 | Example4 | Example5 |
|---|----------|----------|----------|----------|----------|
| Tegafur | 200 mg | 200 mg | 200 mg | 50 mg | 200 mg |
| Indole-3-carbinol | 50 mg | 10 mg | 10 mg | 200 mg | 200 mg |
| (+)-Catechin | 50 mg | 50 mg | 0 | 0 | 0 |
| Myricetin | 0 | 0 | 0 | 100 mg | 0 |
| Kaempferol | 0 | 0 | 0 | 0 | 10 mg |
| Luteolin | 0 | 0 | 200 mg | 0 | 0 |
| Lactose | 400 mg | 400 mg | 400 mg | 400 mg | 400 mg |
| Corn starch | 400 mg | 400 mg | 400 mg | 400 mg | 400 mg |
| Hydroxymethylcellulose | 10 mg | 10 g | 10 g | 10 mg | 10 mg |
| Total | 1110 mg | 1160 mg | 1220 mg | 1160 mg | 1220 mg |

Formulation Example 2: Tablets

A possible pharmaceutical composition for oral use exemplifying but not exhausting the present invention is the following formulation of tablets production:

|  | Example1 | Example2 | Example3 | Example4 | Example5 |
|---|---|---|---|---|---|
| Tegafur | 20 mg | 35mg | 40 mg | 10 mg | 35 mg |
| 13C | 10 mg | 5 mg | 10 mg | 40 mg | 3.5 mg |
| (+)-Catechin | 20 mg | 15 mg | 0 | 0 | 0 |
| Myricetin | 0 | 0 | 0 | 5 mg | 10 mg |
| Kaempferol | 0 | 0 | 0 | 0 | 0 |
| Luteolin | 0 | 0 | 5 mg | 0 | 0 |
| Lactose | 100 mg | 100 mg | 100 mg | 100 mg | 100 mg |
| Crystalline cellulose | 50 mg | 50 mg | 50 mg | 50 mg | 50 mg |
| Magnesium stearate | 10 mg | 10 mg | 10 mg | 10 mg | 10 mg |
| Talc | 10 mg | 10 mg | 10 mg | 10 mg | 10 mg |
| Total | 220 mg | 225 mg | 225 mg | 225 mg | 218.5 mg |

Formulation Example 3: Capsules
A possible pharmaceutical composition for oral use exemplifying but not exhausting the present invention is the following formulation of capsules production:

|  | Example1 | Example2 | Example3 | Example4 | Example5 |
|---|---|---|---|---|---|
| Tegafur | 100 mg | 100 mg | 50 mg | 150 mg | 50 mg |
| I3C | 25 mg | 50 mg | 100 mg | 50 mg | 150 mg |
| (+)-Catechin | 25 mg | 25 mg | 0 | 0 | 0 |
| Myricetin | 0 | 0 | 100 mg | 0 | 50 mg |
| Kaempferol | 0 | 0 | 0 | 50 mg | 0 |
| Luteolin | 0 | 50 mg | 0 | 0 | 0 |
| Lactose | 50 mg | 50 mg | 50 mg | 50 mg | 50 mg |
| Talc | 50 mg | 50 mg | 50 mg | 50 mg | 50 mg |
| Ca stearate | 6 mg | 7 mg | 7 mg | 7 mg | 7 mg |
| Aerosil | 4 mg | 3 mg | 3 mg | 3 mg | 3 mg |
| Total | 260 | 335 mg | 360 mg | 360 mg | 360 mg |

Formulation Example 4: Suppository
A possible pharmaceutical composition for internal use exemplifying but not exhausting the present invention is the following formulation of suppository production:

|  | Example 1 | Example 2 | Example 3 | Example 4 | Example 5 |
|---|---|---|---|---|---|
| Tegafur | 250 mg | 250 mg | 300 mg | 100 mg | 100 mg |
| 13C | 50 mg | 100 mg | 50 mg | 150 mg | 200 mg |
| (+)-Catechin | 100 mg | 0 | 0 | 0 | 100 mg |
| Myrcetin | 0 | 100 mg | 0 | 0 | 0 |
| Kaempferol | 0 | 0 | 100 mg | 0 | 0 |
| Luteolin | 0 | 0 | 0 | 150 mg | 0 |
| Witepsol W-35 | 800 mg | 750 mg | 750mg | 800 mg | 800 mg |

(continued)

|  | Example 1 | Example 2 | Example 3 | Example 4 | Example 5 |
|---|---|---|---|---|---|
| Total | 1200 mg | 1200 mg | 1200 mg | 1200 mg | 1200 mg |

Formulation Example 5: Suspensions

A possible pharmaceutical composition for topical use exemplifying but not exhausting the present invention is the following formulation of suspension production:

|  | Example 1 | Example 2 | Example 3 | Example 4 | Example 5 |
|---|---|---|---|---|---|
| Tegafur | 250 mg | 100 mg | 80 mg | 100 mg | 200 mg |
| 13C | 50 mg | 200 mg | 200 mg | 50 mg | 100 mg |
| (+)-Catechin | 50 mg | 0 | 0 | 0 | 0 |
| Myricetin | 0 | 200 mg | 0 | 0 | 0 |
| Kaempferol | 0 | 0 | 80 mg | 0 | 50 mg |
| Luteolin | 0 | 0 | 0 | 50 mg | 0 |
| Glycerin | 100 mg | 100 mg | 100 mg | 50 mg | 50 mg |
| Disstilled water | 150 mg | 200 mg | 100 mg | 100 mg | 100 mg |
| Total | 600 mg | 800 mg | 560 mg | 350 mg | 500 mg |

Industrial Application

According to the invention, the pharmaceutical composition for treating malignant tumour which contains Tegafur, indole-3-carbinol and one of natural flavonoid derivative, preferably (+)-Catechin, Kaempferol, Myricetin and Luteolin in clinically efficacious amount might be manufactured in a standard pharmaceutical plant.

**Claims**

1. A pharmaceutical composition comprising Tegafur [1-(2-tetrahydrofuryl)-5-fluorouracil] or a pharmaceutically acceptable salt thereof, Indole-3-carbinol and one natural flavonoid derivative, selected from the group of Catechin, Kaempferol, Myricerin or Luteolin.

2. A pharmaceutical composition according to claim 1, wherein natural flavonoid derivative is Catechin.

3. A pharmaceutical composition according to claim 1, wherein natural flavonoid derivative is Kaempferol.

4. A pharmaceutical composition according to claim 1, wherein natural flavonoid derivative is Myricerin.

5. A pharmaceutical composition according to claim 1, wherein natural flavonoid derivative is Luteolin.

6. A pharmaceutical composition according to claim 1, wherein the preferable ratio of the active ingredients Tegafur and Indole-3-carbinolde and one natural flavonoid derivative, selected from the group of Catechin, Kaempferol, Myricerin or Luteolin is from 100:1:1 to 1:100:100.

7. A pharmaceutical composition according to claim 1, wherein the preferable ratio of the active ingredients Tegafur and Indole-3-carbinolde and one natural flavonoid derivative, selected from the group of Catechin, Kaempferol, Myricerin or Luteolin is from 25:1:1 to 1:25:25.

8. A pharmaceutical composition according to claim 1, wherein the preferable ratio of the active ingredients Tegafur and Indole-3-carbinolde and one natural flavonoid derivative, selected from the group of Catechin, Kaempferol, Myricerin or Luteolin is from 5:1:1 to 1:5:5.

9. A pharmaceutical composition according to claim 1, wherein the preferable ratio of the active ingredients Tegafur and Indole-3-carbinolde and one natural flavonoid derivative, selected from the group of Catechin, Kaempferol, Myricerin or Luteolin is from 2:1:1 to 1:2:2.

10. Use of pharmaceutical composition according to any preceding claim as a medicament.

11. A pharmaceutical composition according to claims 1-9 for use in the treatment of a tumour.

12. Use of a pharmaceutical composition according to claims 1-9 for the manufacture of a medicament for administration simultaneously, sequentially or separately for treatment and/or prevention of a tumour.

EP 2 422 848 A1

Europäisches
Patentamt
European
Patent Office
Office européen
des brevets

**EUROPEAN SEARCH REPORT**

Application Number

EP 10 17 3253

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | FAN S ET AL: "BRCA1 and BRCA2 as molecular targets for phytochemicals indole-3-carbinol and genistein in breast and prostate cancer cells" BRITISH JOURNAL OF CANCER, vol. 94, no. 3, February 2006 (2006-02), pages 407-426, XP002506760 ISSN: 0007-0920 * abstract; figure 4g * * page 416, left-hand column, paragraph 1 * | 1-12 | INV. A61P35/00 A61K31/404 A61K31/513 A61K31/353 |
| A | POUGET C ET AL: "FLAVONOIDS: STRUCTURAL REQUIREMENTS FOR ANTIPROLIFERATIVE ACTIVITY ON BREAST CANCER CELLS" BIOORGANIC & MEDICINAL CHEMISTRY LETTERS, PERGAMON, ELSEVIER SCIENCE, GB, vol. 11, no. 24, 1 January 2001 (2001-01-01), pages 3095-3097, XP009058137 ISSN: 0960-894X * page 3097, left-hand column, paragraph 3 - right-hand column, paragraph 1 * | 1-12 | |
| A | HOWELLS LYNNE M ET AL: "Predicting the physiological relevance of in vitro cancer preventive activities of phytochemicals." ACTA PHARMACOLOGICA SINICA SEP 2007, vol. 28, no. 9, September 2007 (2007-09), pages 1274-1304, XP002506708 ISSN: 1671-4083 * page 1279, right-hand column, paragraph 3 - page 1280, left-hand column, paragraph 1 * | 1-12 | TECHNICAL FIELDS SEARCHED (IPC) A61K A61P |

-/--

The present search report has been drawn up for all claims

| Place of search | Date of completion of the search | Examiner |
|---|---|---|
| Munich | 9 November 2010 | Herrera, Suzanne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

9

Europäisches
Patentamt
European
Patent Office
Office européen
des brevets

# EUROPEAN SEARCH REPORT

**Application Number**

EP 10 17 3253

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DEORUKHKAR A ET AL: "Back to basics: How natural products can provide the basis for new therapeutics" EXPERT OPINION ON INVESTIGATIONAL DRUGS 200711 GB, vol. 16, no. 11, November 2007 (2007-11), pages 1753-1773, XP002506709 ISSN: 1354-3784 * page 1763, right-hand column, paragraph 3 - page 1764, left-hand column, paragraph 2 * | 1-12 | |
| A | SCHMOLL H-J: "DIHYDROPYRIMIDINE DEHYDROGENASE INHIBITION AS A STRATEGY FOR THE ORAL ADMINISTRATION OF 5-FLUOROURACIL: UTILITY IN THE TREATMENT OF ADVANCED COLORECTAL CANCER" ANTI-CANCER DRUGS, RAPID COMMUNICATIONS, OXFORD, vol. 14, no. 9, 1 January 2003 (2003-01-01), pages 695-702, XP009062217 ISSN: 0959-4973 | 1-12 | |

TECHNICAL FIELDS
SEARCHED      (IPC)

The present search report has been drawn up for all claims

| Place of search | Date of completion of the search | Examiner |
|---|---|---|
| Munich | 9 November 2010 | Herrera, Suzanne |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 1168391 A
- US 4328229 A
- US 2007099844 A
- WO 2005105086 A

**Non-patent literature cited in the description**

- *Cancer Research,* 1995, vol. 1, 839-845
- **SARKAR FH ; LI Y.** Indole-3-carbinol and prostate cancer. *The Journal of Nutrition,* 2004, vol. 134 (12), 3493S-3498S
- *Cancer research,* 2001, 118-125